# EUROPEAN PATENT SPECIFICATION

(11) **EP 1 542 753 B1**
(45) Date of publication and mention of the grant of the patent: **07.02.2007**
(21) Application number: 03793261.3
(22) Date of filing: 21.08.2003
(51) Int. Cl.: A61M 15/00, A61K 9/00, A61K 9/12

(54) **PHARMACEUTICAL COMPOSITIONS**
PHARMAZEUTISCHE ZUSAMMENSETZUNGEN
COMPOSITIONS PHARMACEUTIQUES

(30) Priority: 23.08.2002 US 405563 P
(43) Date of publication of application: 22.06.2005
(73) Proprietor: SCHERING CORPORATION, Kenilworth, NJ 07033-0530 (US)
(72) Inventor: SHARPE, Stefan, A., Jersey City, NJ 07302 (US); SEQUEIRA, Joel, A., Edison, NJ 08820 (US)
(74) Representative: Naylor, Kathryn May
(86) International application number: PCT/US2003/026272
(87) International publication number: WO 2004/018025

(56) References cited:
- WO-A-01/64274
- WO-A-02/30394
- WO-A-03/020253
- US-A- 5 474 759

## Description

The present invention is directed to aerosol suspension formulations which are free of chlorofluorocarbons (CFC's). More specifically, the present invention is directed to formulations that are substantially free of CFC's and formulations that have particular utility in medicinal applications, especially in metered dose inhalers (MDI's). An inhaler according to the preamble of claim 1 is disclosed in WO-A-01/64274.

Metered dose inhalers have proven to be effective oral and nasal delivery systems that have been used extensively for delivering bronchodilating and steroidal compounds to asthmatics, as well as delivering other compounds such as pentamidine and non-bronchodilator anti-inflammatory drugs. The rapid onset of activity of compounds administered in this manner and the absence of any significant side effects have resulted in a large number of compounds being formulated for administration via this route. Typically, the drug is delivered to the patient by a propellant system generally comprising one or more propellants which have the appropriate vapor pressure and which are suitable for oral or nasal administration. The more preferred propellant systems typically comprise CFC propellant 11, CFC propellant 12, CFC propellant 114 or mixtures thereof. Often the vapor pressure of the propellant systems is adjusted by admixing a less volatile liquid excipient with the propellant.

However, propellants CFC 11, CFC 12 and CFC 114 belong to a class of compounds known as chlorofluorocarbons, which have been linked to the depletion of ozone in the atmosphere. It has been postulated that ozone blocks certain harmful UV rays and thus a decrease in the atmospheric ozone content will result in an increase in the incidence of skin cancer. In the 1970's certain steps were taken to reduce the CFC emissions from aerosols. Other propellants, such as hydrocarbons, were used, or the product was delivered in a different manner. Because CFC usage in medicinal applications is relatively low, i.e. less than 1% of total CFC emissions, and because of the health benefits associated with metered dose inhalers, steps were not taken at that time to restrict the use of CFC propellants in metered dose inhalers.

However, continuing and more sophisticated ozone measurements have indicated that the earlier restrictions in CFC usage were insufficient and that additional, significant steps should be taken to drastically reduce CFC emissions. Recommendations have been made that CFC production be virtually discontinued. As a result, it may not be possible to continue to use CFC propellants in the intermediate and long term. While some efforts have been made to use non-pressurized metered dose inhalers, many of these devices have not been completely successful. Some of the performance issues related to these are: delivery of uniform doses, mechanical complexity, provision of the required doses per unit of an aerosol container, compliance with stringent regulatory standards, and difficulty for individuals to utilize because they are bulky and/or cumbersome for patient use, particularly when patient has an acute need for the medication.

As a result, there is a need for CFC-free pressurized aerosol formulations, such as metered dose inhalers, which are substantially free of CFC's. Non-CFC propellant systems must meet several criteria for pressurized metered dose inhalers. They must be non-toxic, stable and non-reactive with the medicament and the other major components in the valve/actuator. One propellant which has been found to be suitable is CF₃CHFAF₃, also known as HFA 227, HFC 227 or 1,1,1,2,3,3,3 heptafluoropropane, hereinafter HFA 227. However, certain physical properties, i.e., polarity and solubility of HFA 227 differ from those of commonly used CFC propellants. Commonly used surfactants may be insoluble in HFA 227. Moreover, where the medicament is to be delivered as a solution, the medicament may not be readily soluble in this propellant. The polarity difference between HFA 227 and the previously used CFC propellants may result in a different delivery of the medicament when HFA 227 replaces a CFC propellant. Another such non-chlorofluorocarbon propellant is Hydrofluorocarbon 134a, also known as 1,1,1,2-tetrafluoroethane or HFA 134a, hereinafter HFA 134a.

Prior art formulations containing mometasone in combination with HFA 227 in a metered dose inhaler utilize ethanol to suspend the mometasone in a crystalline state in combination with the propellant. These formulations have improved stability over time.

The specific combinations noted above may not provide the desired solubility, stability, low toxicity, exact dosage, correct particle size (if suspension) and/or compatibility with commonly used valve assemblies of metered dose inhalers. Accordingly, there exists a need for CFC free formulations for the treatment of asthma, and processes for producing the same, that do not suffer from the aforementioned shortcomings.

### SUMMARY OF THE INVENTION

Accordingly, the present invention is directed to a metered dose inhaler containing an aerosol suspension formulation for inhalation, said aerosol suspension formulation for inhalation comprising: an effective amount of mometasone furoate, a surfactant and a chlorofluorocarbon free propellant, preferably HFA 227 or HFA 134a, wherein the formulation is substantially free of a carrier.

The present invention is also directed to a process for producing an aerosol suspension formulation for inhalation, said aerosol suspension formulation for inhalation comprising: an effective amount of mometasone furoate and a non-chlorofluorocarbon based propellant; wherein the formulation is free of a bulking agent, comprising the steps of a) mixing a dry powder blend of micronized mometasone with a dry powder surfactant to form a uniform mixture; b) filling said mixture into a metered dose inhaler canister; c) crimping said canister with a metering valve; and d) filling said canister with a non-chlorofluorocarbon based propellant, and the products produced thereby.

The present invention is also directed to a metered dose inhaler containing an aerosol suspension formulation for inhalation, said aerosol suspension formulation for inhalation comprising: an effective amount of mometasone furoate; a dry powder surfactant; and HFA 227; wherein the mometasone furoate is present in an amount of about 400 µg to about 50 µg, wherein the formulation is free of additional excipients, and wherein the metered dose inhaler emits a dose having uniform drug content upon actuation of the metered dose inhaler.

The present invention is also directed to a metered dose inhaler containing an aerosol suspension formulation for inhalation, said aerosol suspension formulation for inhalation comprising: an effective amount of mometasone furoate and HFA 227; wherein the mometasone furoate is present in an amount of about 400 µg about 50 µg, wherein the percent of the fine particles dispensed upon actuation of the metered dose inhaler is about 55% to about 85%, and wherein said fine particles have a particle size of less than about 4.7µm.

### DETAILED DESCRIPTION OF THE INVENTION

Mometasone furoate, the active component of ELOCON® lotion, cream, and ointment, and NASONEX nasal spray, is an anti-inflammatory corticosteroid having the chemical name, 9,21-Dichloro-11(beta), 17-dihydroxy-16(alpha)-methylpregna-1,4-diene-3,20-dione 17-(2 furoate). It is practically insoluble in water; slightly soluble in methanol, ethanol, and isopropanol; soluble in acetone and chloroform; and freely soluble in tetrahydrofuran. Its partition coefficient between octanol and water is greater than 5000. Mometasone can exist in various hydrated and crystalline forms. This product is available from Schering-Plough Corporation, Kenilworth, New Jersey. The present invention is of particular utility where the medicament is mometasone furoate, or end salts, enantiomers and clathrates thereof.

The mometasone can be dosed at, for example, about 50 µg of mometasone furoate per dose, or about 100 µg of mometasone furoate per dose, or about 200 µg mometasone furoate per dose, or about 400 µg of mometasone furoate per dose.

Additional active ingredients may be employed in the formulations of the present invention. For instance, formoterol fumarate is a selective beta ₂-adrenergic bronchodilator that can be added to the formulations of present invention. Formoterol fumarate can exist in various hydrated, crystalline, and enantiomeric forms, e.g., as a monohydrate. This product is available commercially from Schering-Plough Corporation, Kenilworth, New Jersey and Novartis Corporation, East Hanover, New Jersey.

Propellant-based pharmaceutical aerosol formulations in the art typically use a mixture of liquid chlorofluorocarbons as the propellant, although many others use a single propellant. As is known in the art, the propellant serves as a vehicle for both the active ingredients and excipients. Fluorotrichloromethane, dichlorodifluoromethane and dichlorotetrafluoroethane are the most commonly used propellants in aerosol formulations for administration by inhalation. Such chlorofluorocarbons (CFC's), however, have been implicated in the destruction of the ozone layer and their production is being phased out. HFA 134a and HFA 227 are said to be less harmful to the ozone than many chlorofluorocarbon propellants, and both either individually or in combination are considered to be within the scope of the present invention. However, conventional chloroflourocarbons, or mixtures thereof, may also be used as propellants for the formulations of the present invention.

As is known to one of skill in the art, a carrier and/or bulking agent is an inert substance in which or on to which the active drug ingredient(s) and excipient(s) if present are dispersed. When the formulations of the present invention utilize HFA 227 as the propellant, it has been surprisingly found that a carrier is not necessary. Accordingly there is disclosed a metered dose inhaler containing an aerosol suspension formulation for inhalation, said aerosol suspension formulation for inhalation comprising: an effective amount of mometasone furoate and HFA 227, wherein the formulation is substantially free of a carrier. The processes for producing the formulations of the present invention preferably utilize HFA 227 or HFA 134a, or a combination thereof, in combination with mometasone furoate and dry powder surfactant.

The active ingredients may be put into the containers housing the formulation as follows: the container that houses the medication can be filled with medicine, ethanol and a surfactant in single or multiple steps, preferably in a single step. Similarly, the propellant or mixture of propellants may be added to the container in the same or in multiple steps.

Formulations of the invention are made according to procedures customary in the art for other aerosol compositions. Typically in a 2-stage filling method all the ingredients except the propellant are mixed in a vessel. The required amount of the above mixture is metered into the individual cans. The valve is crimped onto the cans and then the appropriate amount of propellant is added through the valve. In a 1-stage filling method, all ingredients including the propellant are mixed and introduced into a vessel. The valves are crimped onto the cans and the entire formulation is then metered into the can. Alternately in a cold filling method, a compounding vessel is chilled to temperatures below the boiling point of the propellant, all the ingredients including the chilled propellant (below its boiling temperature) are added to the vessel. The required amount of the formulation is metered into the can and the valve is then crimped onto the can.

The formulations of the present invention may be filled into the aerosol containers using conventional filling equipment. Since HFA 227 and HFA 134a may not be compatible with all elastomeric compounds currently utilized in present aerosol valve assemblies, it may be necessary to substitute other materials, such as white buna rubber, or to utilize excipients and optionally surfactants which mitigate the adverse effects of HFA 227 or 134a on the valve components. Suspensions of the present invention preferably may be prepared by either the pressure filling or cold filling procedures known in the art.

Depending on the particular application, the container may be charged with a predetermined quantity of formulation for single or multiple dosing. Typically, the container is sized for multiple-dosing, and, therefore it is very important that the formulation delivered is substantially uniform for each dosing. For example, where the formulation is for bronchodilation, the container typically is charged with a sufficient quantity of the formulation for 120 or 200 actuations.

Suitable suspensions may be screened in part by observing several physical properties of the formulation, i.e. the rate of particle agglomeration, the size of the agglomerates and the rate of particulate creaming/settling and comparing these to an acceptable standard. Such, suitable solutions may be screened/evaluated by measuring the solubility of the medicament over the entire recommended storage temperature range.

For metered dose inhalers, suspensions may be particularly preferred for efficacy and stability considerations. Those skilled in the art may choose to add one or more preservative, buffer, antioxidant, sweetener and/or flavors or other taste masking agents depending upon the characteristics of the formulation.

The available metering valve delivery volumes range from about 25 to about 100 microliters per actuation, while the amounts of drug substance required in a dose for treating a particular condition is generally about 10 to about 500 micrograms per valve actuation. These two factors combined pose limitations that dictate the points within the foregoing ethanol parameters for a given formulation. The determination of such amounts is within the skill of workers in this art.

In formulations of the present invention which are suitable for treating lower respiratory system disorders such as asthma, at least a substantial portion of the drug is present as suspended particles having respirable sizes, e.g., about 0.5 to about 10 micrometers in their largest dimension. In formulations which are suitable for treating upper respiratory system disorders such as rhinitis, somewhat larger drug particles may be permissible, but the foregoing size range remains preferred. Where the active compound forms a suspension, the particle size should be relatively uniform, with substantially all the particles preferably ranging between about 0.1-25 microns, preferably 0.5-10 microns, more preferably 1-5 microns. Particles larger than 10 microns may be held up in the oropharyngeal cavity, while particles smaller than about 0.5 micron preferably are not utilized, since they would be more likely to be exhaled and, therefore, not reach the lungs of the patient.

Another aspect of the present invention comprises novel formulations comprising a dispersion system of a well mixed binary blend of a drug substance powder mometasone furoate dispersed with a second powder-surfactant, such as, for example lecithin, stearic acid, palmitic acid, magnesium stearate, magnesium palmitate, magnesium laureate and other suitable dry powder blend surfactants as are known to one of skill in the art.

The dry blend may be mixed for example in a Turbula Mixer T2C for about 5 minutes, or for such amount of time is known to one of skill in the art to achieve a uniform blend of the powders. This dispersion system is metered individually into each inhaler can with a powder filling instrument, such as for example by an Autodose Powdernium - One Too Many System, into 15 mL aluminum teflon coated (FPT - fluorinated ethylene copolymer) or other polymer coated, cans. The cans can then be crimped with 63 microliter valves or the like and filled with HFA-227 or HFA-134a propellant using propellant filling equipment, such as, for example, a Pamasol Model P2008/012. The cans filled with the suspension product are thereafter sonicated by a sonicator, such as, for example, a Branson 5210 sonicator for about 5 minutes as is known to one in the art.

These particular formulations allow for the manufacture of a drug substance in an MDI that exhibits a consistent Drug Dose Uniformity (DDU) without the use of additional excipients and/or additives. The use of this type of dry 2-step filling procedure precludes the possibility of crystal growth of the active ingredients during the filling process and assures a consistent particle size distribution in the product filled during the beginning, middle and end of the filling process. This formulation and filling process assure adequate dispersion of the particles in the suspending medium HFA-227, absence of crystal growth, absence of caking and adequate DDU upon delivery of the dose.

Certain aspects of the invention are further described in the following examples. In the examples, "percent" indicates weight percentage unless the context clearly indicates otherwise. The examples below further describe the present invention.

The following dry powder blend samples were prepared.

### Example 1

| **Table 1. Dry Powder Blends of Mometasone Furoate (99.9%) & Lecithin (0.1%; 0.01% and 0.02%)*** | | | |
|---|---|---|---|
| **Mometasone Furoate (mg)** | **Lecithin (mg)** | **Total Weight of Blend (mg)** | **Weight Per Can (mg)** |
| 616.0 | 0.686 | 616.7 | 12.25 |
| 621.0 | 0.070 | 621.07 | 11.35 |
| 621.0 | 0.144 | 621.12 | 11.45 |
| *: All weights presented on the w/w basis in the binary blend. | | | |

To prepare, directly mix a dry powder blend of the mometasone furoate, formoterol fumarate and lecithin in a Turbula mixer for about 5 minutes in the above identified amounts. Thereafter, meter the mixture into the 15 mL canister manually or using an Autodose Powdemium powder filling instrument or the like. Thereafter, crimp with a 63 microliter valve and add the propellant up to about 10 g/can. Then, sonicate for 5 minutes.

### Example 2

| **Table 2. MDI Formulation Blends of Mometasone Furoate Lecithin and HFA- 227*** | | |
|---|---|---|
| **Mometasone Furcate (%)** | **Lecithin (%)** | **HFA-227 (%)** |
| 0.1 | 0.01 | 99.89 |
| 0.1 | 0.001 | 99.89 |
| 0.1 | 0.002 | 99.89 |
| *: All weights presented on the w/w basis in the finished product. | | |

Table 2 describes the various amounts of the active ingredient and surfactant when combined with HFA-227 in the finished metered dose inhaler canister.

A finer particle size distribution of the mometasone furoate improves the fine particle fraction of the formulation exiting the inhaler upon actuation of the metered dose inhaler. Indeed, with a MDI using mometasone furoate with a finer grade of mometasone furoate, there is a substantial decrease in the percent of change in fine particle size under typical temperature and relative humidity cycling conditions. This results in an increase in the fine particle fraction with regards to the mometasone, and thus improved drug delivery of the mometasone. Thus, it has been found that when a finer particle size grade of the drug substance is used, a product is produced which has suspended drug particles which do not exhibit particle growth with time and temperature. The aerodynamic particle size distribution is well within the range of a typical efficacious topical lung medication, e.g., greater than 50% of the particles are less than 4.7 microns. It also shows no significant particle growth with time and temperature.

There is a rank order correlation of the quality of the product with a decrease in the size range of the corresponding drug substance suspended in the product. It was determined that drug substance containing a high proportion of large crystals that are greater than 5 to 10 microns produces a product with an aerodynamic particle size distribution that is outside the range of a typical efficacious topical lung medication. The product containing coarser drug product also shows unacceptable particle growth with time and temperature.

The size of the suspended mometasone furoate drug contained in the drug product may be controlled in various ways. The drug substance may be more efficiently milled prior to product batch manufacture. This could include reducing the micronization feed rate, employing centrifugal classification to remove larger particles and increasing the number of cycles the material is fed into the micronizer, e.g., double micronizing. Alternatively, the drug substance may be spray dried prior to product batch manufacture, for example, by super critical fluid technology, to create uniformly small drug substance particles. Further the method of manufacture can be modified, e.g., by reducing the temperature of batch manufacture, reducing the level of alcohol used to prepare the drug concentrate, or reducing the homogenization time. Finally, other processes of controlling drug substance particle size that are known in the art, e.g., using surfactants or other particle size growth retardation approaches may also be used.

In the case of the oral MDI containing mometasone furoate, an example of an acceptable product profile for the 100 µg /actuation strength, using an Andersen Cascade Impactor and 1-liter entry port, is given below. It should be noted that the data is based on two actuations of the metered dose inhaler.

| **Table 3. Mometasone Furoate with 0.01% Lecithin - HFA-227 Formulation** | | | |
|---|---|---|---|
| **ACI** | **Amount per Group (micrograms)** | | |
| | **High** | **Low** | **Average** |
| Group I - (Entry Port + Stage 0) | 4.8 | 4.1 | 4.3 |
| Group II- (Stage 1 + Stage 2) | 5.8 | 5.4 | 5.6 |
| Group III- (Stage 3 + Stage 4) | 61.6 | 56.7 | 59.1 |
| Group IV - (Stage 5 - Filter) | 18.0 | 14.8 | 16.7 |
| % Fine Particles | 79.5 | 71.5 | 75.8 |

The percentage of particles in Group I ranges from about 4.1 % to about 4.8%. The percentage of particles in Group II ranges from about 5.4% to about 5.8%. The percentage of particles in Group III to the filter should preferably be in a range of about 55% to about 90% where the fine particles have a particle size of less than about 4.7µm, preferably 60 to 80%, or about 75%, or about 85%, and about 88.3% based upon data from above table. Finally, the percentage of particles in Group IV ranges from about 14.8% o about 18%.

| **Table 4. Mometasone Furoate with 0.02% Lecithin - HFA-227 Formulation** | | | |
|---|---|---|---|
| **ACI** | **Amount per Group (micrograms)** | | |
| | **High** | **Low** | **Average** |
| Group I - (Entry Port + Stage 0) | 5.2 | 4.6 | 4.9 |
| Group II- (Stage 1 + Stage 2) | 6.5 | 5.2 | 6.0 |
| Group III- (Stage 3 + Stage 4) | 57.4 | 56.7 | 57.0 |
| Group IV - (Stage 5 - Filter) | 14.2 | 13.1 | 13.7 |
| % Fine Particles | 70.9 | 70.6 | 70.7 |

The percentage of particles in Group I ranges from about 4.6% to about 5.2%. The percentage of particles in Group II ranges from about 5.2% to about 6.5%. The percentage of particles in Group III to the filter should preferably be in a range of about 55% to about 90% where the fine particles have a particle size of less than about 4.7µm, preferably 65% to 80%, or about 75%, or about 80%, or about 85%, and about 87.7% to about 86% based upon data from above table. Finally, the percentage of particles in Group IV ranges from about 13.1% to about 14.2%.

| **Table 5. Mometasone Furoate with 0.1% Lecithin - HFA-227 Formulation** | | | |
|---|---|---|---|
| **ACI** | **Amount per Group (micrograms)** | | |
| | **High** | **Low** | **Average** |
| Group I - (Entry Port + Stage 0) | 5.9 | 4.7 | 5.4 |
| Group II- (Stage 1 + Stage 2) | 7.1 | 6.6 | 6.9 |
| Group III- (Stage 3 + Stage 4) | 61.1 | 53.9 | 56.7 |
| Group IV - (Stage 5 - Filter) | 15.5 | 13.1 | 14.6 |
| % Fine Particles | 76.4 | 67.0 | 71.3 |

The percentage of particles in Group I ranges from about 4.7% to about 5.9%. The percentage of particles in Group II ranges from about 6.6% to about 7.1 %. The percentage of particles in Group III to the filter should preferably be in a range of about 55% to about 90% where the fine particles have a particle size of less than about 4.7µm, preferably 65% to 80%, or about 75%, or about 80%, or about 85%, and about 85.5% based upon data from above table. Finally, the percentage of particles in Group IV ranges from about 15.5% to about 13.1%.

It will of course be apparent to one of skill in the art that the data in Tables 3 to 5 may change depending upon the size of the entry port of the Andersen Cascade Impactor.

The foregoing descriptions of various embodiments of the invention are representative of various aspects of the invention, and are not intended to be exhaustive or limiting to the precise forms disclosed. Many modifications and variations undoubtedly will occur to those having skill in the art. It is intended that the scope of the invention shall be fully defined solely by the appended claims.

## Claims

1. A metered dose inhaler containing an aerosol suspension formulation for inhalation, said aerosol suspension formulation for inhalation comprising: an effective amount of mometasone furoate and HFA 227, **characterized in that** the formulation comprises a dry powder surfactant and is substantially free of a carrier.

2. The metered dose inhaler containing an aerosol suspension formulation for inhalation according to claim 1, wherein the mometasone furoate is present in an amount of about 50 µg to about 400 µg.

3. The metered dose inhaler containing an aerosol suspension formulation for inhalation according to claim 2, wherein the mometasone furoate is present in an amount of about 100 µg.

4. The metered dose inhaler containing an aerosol suspension formulation for inhalation according to claim 2, wherein the mometasone furoate is present in an amount of about 200 µg.

5. The metered dose inhaler containing an aerosol suspension formulation for inhalation according to claim 2, wherein the mometasone furoate is present in an amount of about 400 µg.

6. The metered dose inhaler containing an aerosol suspension formulation for inhalation according to claim 1, wherein the dry powder surfactant is selected from the group consisting of lecithin, stearic acid, palmitic acid, magnesium stearate, magnesium palmitate, and magnesium laureate.

7. The metered dose inhaler containing an aerosol suspension formulation for inhalation according to claim 1, wherein the formulation is free of additional excipients, and wherein the metered dose inhaler emits a dose having uniform drug content upon actuation of the metered dose inhaler.

8. The metered dose inhaler containing an aerosol suspension formulation for inhalation according to claim 1, wherein the percent of the fine particles dispensed upon actuation of the metered dose inhaler is about 55% to about 85%, and wherein said fine particles have a particle size of less than about 4.7µm.

9. The metered dose inhaler according to claim 8, wherein the percent of the fine particles dispensed upon actuation of the metered dose inhaler is about 65% to about 80%, and wherein said fine particles have a particle size of less than about 4.7µm.

10. A process for producing an aerosol suspension formulation for inhalation, said aerosol suspension formulation for inhalation comprising:
an effective amount of mometasone furoate and a non-chlorofluorocarbon based propellant; wherein the formulation is free of a carrier, comprising the steps of:
a) mixing a dry powder blend of micronized mometasone with a dry powder surfactant to form a uniform mixture;
b) filling said mixture into a metered dose inhaler canister;
c) crimping said canister with a metering valve; and
d) filling said canister with a non-chlorofluorocarbon based propellant.

11. The process according to claim 10, wherein the dry powder surfactant is selected from the group consisting of lecithin, stearic acid, palmitic acid, magnesium stearate, magnesium palmitate and magnesium laureate.

12. The process according to claim 10, wherein the non-chlorofluorocarbon based propellant is HFA 227.

13. The product produced by the process of claim 10.

14. The product of claim 13, wherein the mometasone furoate is present in an amount of about 50 µg to about 400 µg.

15. The product of claim 14, wherein the mometasone furoate is present in an amount of about 100 µg.

16. The product of claim 14, wherein the mometasone furoate is present in an amount of about 200 µg.

17. The product of claim 14, wherein the mometasone furoate is present in an amount of about 400 µg.

18. The product of claim 13, wherein the formulation is free of additional excipients, and wherein the metered dose inhaler emits a dose having uniform drug content upon actuation of the metered dose inhaler.

19. The product of claim 13, wherein the percent of the fine particles dispensed upon actuation of the metered dose inhaler is about 55% to about 85%, and wherein said fine particles have a particle size of less than about 4.7µm.

20. The product of claim 19, wherein the percent of the fine particles dispensed upon actuation of the metered dose inhaler is about 65% to about 80%, and wherein said fine particles have a particle size of less than about 4.7µm.

## Patentansprüche

1. Dosierinhaliergerät, das eine Aerosol-Suspensions-Formulierung zur Inhalation enthält, wobei die Aerosol-Suspensions-Formulierung zur Inhalation eine wirksame Menge von Mometasonfuroat und HFA 227 enthält, **dadurch gekennzeichnet, dass** die Formulierung ein Trockenpulvertensid enthält und im wesentlichen frei von einem Träger ist.

2. Dosierinhaliergerät, das eine Aerosol-Suspensions-Formulierung zur Inhalation enthält, nach Anspruch 1, bei dem das Mometasonfuroat in einer Menge von etwa 50 µg bis etwa 400 µg vorhanden ist.

3. Dosierinhaliergerät, das eine Aerosol-Suspensions-Formulierung zur Inhalation enthält, nach Anspruch 2, bei dem das Mometasonfuroat in einer Menge von etwa 100 µg vorhanden ist.

4. Dosierinhaliergerät, das eine Aerosol-Suspensions-Formulierung zur Inhalation enthält, gemäß Anspruch 2, bei dem das Mometasonfuroat in einer Menge von etwa 200 µg vorhanden ist.

5. Dosierinhaliergerät, das eine Aerosol-Suspensions-Formulierung zur Inhalation enthält, nach Anspruch 2, bei dem das Mometasonfuroat in einer Menge von etwa 400 µg vorhanden ist.

6. Dosierinhaliergerät, das eine Aerosol-Suspensions-Formulierung zur Inhalation enthält, nach Anspruch 1, bei dem das Trockenpulvertensid ausgewählt ist aus der Gruppe bestehend aus Lecithin, Stearinsäure, Palmitinsäure, Magnesiumstearat, Magnesiumpalmitat und Magnesiumlaureat.

7. Dosierinhaliergerät, das eine Aerosol-Suspensions-Formulierung zur Inhalation enthält, nach Anspruch 1, bei dem die Formulierung frei von zusätzlichen Hilfsmitteln ist und bei dem das Dosierinhaliergerät bei Betätigung des Dosierinhaliergeräts eine Dosis mit gleichförmigem Wirkstoffgehalt abgibt.

8. Dosierinhaliergerät, das eine Aerosol-Suspensions-Formulierung zur Inhalation enthält, nach Anspruch 1, bei dem der Prozentsatz der feinen Teilchen, die bei Betätigung des Dosierinhaliergeräts abgegeben werden, etwa 55 % bis etwa 85 % beträgt und bei dem die feinen Teilchen eine Teilchengröße von weniger als etwa 4,7 µm aufweisen.

9. Dosierinhaliergerät nach Anspruch 8, bei dem der Prozentsatz der feinen Teilchen, die bei Betätigung des Dosierinhaliergeräts abgegeben werden, etwa 65 % bis etwa 80 % beträgt und bei dem die feinen Teilchen eine Teilchengröße von weniger als etwa 4,7 µm aufweisen.

10. Verfahren zur Herstellung einer Aerosol-Suspensions-Formulierung zur Inhalation, wobei die Aerosol-Suspensions-Formulierung zur Inhalation eine wirksame Menge von Mometasonfuroat und ein nicht-Chlorfluorkohlenstoff-basierendes Treibmittel enthält, und wobei die Formulierung frei von einem Träger ist, bei dem
a) ein Trockenpulvergemisch von mikronisiertem Mometason mit einem Trockenpulvertensid gemischt wird, um eine gleichförmige Mischung zu bilden;
b) die Mischung in einen Dosierinhaliergeräte-Kanister gefüllt wird,
c) auf den Kanister ein Dosierventil aufgepresst wird; und
d) der Kanister mit einem nicht-Chlorfluorkohlenstoffbasierenden Treibmittel gefüllt wird.

11. Verfahren nach Anspruch 10, bei dem das Trockenpulvertensid ausgewählt ist aus der Gruppe bestehend aus Lecithin, Stearinsäure, Palmitinsäure, Magnesiumstearat, Magnesiumpalmitat und Magnesiumlaureat.

12. Verfahren nach Anspruch 10, bei dem das nicht-Chlorfluorkohlenstoff-basierende Treibmittel HFA 227 ist.

13. Produkt, das nach dem Verfahren gemäß Anspruch 10 hergestellt worden ist.

14. Produkt nach Anspruch 13, bei dem das Mometasonfuroat in einer Menge von etwa 50 µg bis etwa 400 µg vorhanden ist.

15. Produkt nach Anspruch 14, bei dem das Mometasonfuroat in einer Menge von etwa 100 µg vorhanden ist.

16. Produkt nach Anspruch 14, bei dem das Mometasonfuroat in einer Menge von etwa 200 µg vorhanden ist.

17. Produkt nach Anspruch 14, bei dem das Mometasonfuroat in einer Menge von etwa 400 µg vorhanden ist.

18. Produkt nach Anspruch 13, bei dem die Formulierung frei von zusätzlichen Hilfsmitteln ist und bei dem das Dosierinhaliergerät bei Betätigung des Dosierinhaliergeräts eine Dosis mit gleichförmigem Wirkstoffgehalt abgibt.

19. Produkt nach Anspruch 13, bei dem der Prozentsatz der feinen Teilchen, die bei Betätigung des Dosierinhaliergeräts abgegeben werden, etwa 55 % bis etwa 85 % beträgt und bei dem die feinen Teilchen eine Teilchengröße von weniger als etwa 4,7 µm aufweisen.

20. Produkt nach Anspruch 19, bei dem der Prozentsatz der feinen Teilchen, die bei Betätigung des Dosierinhaliergeräts abgegeben werden, etwa 65 % bis etwa 80 % beträgt und bei dem die feinen Teilchen eine Teilchengröße von weniger als etwa 4,7 µm aufweisen.

## Revendications

1. Inhalateur-doseur contenant une formulation de type suspension en aérosol pour inhalation, laquelle formulation de type suspension en aérosol pour inhalation comprend du furoate de mométasone, en une quantité efficace, et du HFA 227, **caractérisé en ce que** ladite formulation comprend un tensioactif en poudre sèche et ne contient pratiquement pas de véhicule.

2. Inhalateur-doseur contenant une formulation de type suspension en aérosol pour inhalation, conforme à la revendication 1, dans lequel le furoate de mométasone se trouve en une quantité d'à peu près 50 à 400 µg.

3. Inhalateur-doseur contenant une formulation de type suspension en aérosol pour inhalation, conforme à la revendication 2, dans lequel le furoate de mométasone se trouve en une quantité d'à peu près 100 µg.

4. Inhalateur-doseur contenant une formulation de type suspension en aérosol pour inhalation, conforme à la revendication 2, dans lequel le furoate de mométasone se trouve en une quantité d'à peu près 200 µg.

5. Inhalateur-doseur contenant une formulation de type suspension en aérosol pour inhalation, conforme à la revendication 2, dans lequel le furoate de mométasone se trouve en une quantité d'à peu près 400 µg.

6. Inhalateur-doseur contenant une formulation de type suspension en aérosol pour inhalation, conforme à la revendication 1, dans lequel le tensioactif en poudre sèche est choisi dans l'ensemble formé par de la lécithine, de l'acide stéarique, de l'acide palmitique, du stéarate de magnésium, du palmitate de magnésium et du laurate de magnésium.

7. Inhalateur-doseur contenant une formulation de type suspension en aérosol pour inhalation, conforme à la revendication 1, dans lequel ladite formulation ne contient pas d'excipients supplémentaires, et lequel inhalateur-doseur émet, après sa mise en action, une dose contenant une quantité régulière de médicament.

8. Inhalateur-doseur contenant une formulation de type suspension en aérosol pour inhalation, conforme à la revendication 1, avec lequel la proportion des fines particules dispensées après mise en action de l'inhalateur-doseur vaut à peu près de 55 à 85 %, et dans lequel lesdites fines particules ont une taille de moins d'environ 4,7 µm.

9. Inhalateur-doseur conforme à la revendication 8, avec lequel la proportion des fines particules dispensées après mise en action de l'inhalateur-doseur vaut à peu près de 65 à 80 %, et dans lequel lesdites fines particules ont une taille de moins d'environ 4,7 µm.

10. Procédé de production d'une formulation de type suspension en aérosol pour inhalation, laquelle formulation de type suspension en aérosol pour inhalation comprend du furoate de mométasone, en une quantité efficace, et un agent propulseur qui n'est pas à base de chlorofluorocarbone, et laquelle formulation ne contient pas de véhicule, lequel procédé comporte les étapes suivantes :
a) mélanger une forme en poudre sèche de mométasone micronisée avec un tensioactif en poudre sèche, pour en faire un mélange homogène,
b) mettre de ce mélange dans une cartouche d'inhalateur-doseur,
c) fixer par sertissage une valve doseuse sur ladite cartouche,
d) et mettre dans cette cartouche un agent propulseur qui n'est pas à base de chlorofluorocarbone.

11. Procédé conforme à la revendication 10, dans lequel le tensioactif en poudre sèche est choisi dans l'ensemble formé par de la lécithine, de l'acide stéarique, de l'acide palmitique, du stéarate de magnésium, du palmitate de magnésium et du laurate de magnésium.

12. Procédé conforme à la revendication 10, dans lequel l'agent propulseur qui n'est pas à base de chlorofluorocarbone est du HFA 227.

13. Produit obtenu par un procédé conforme à la revendication 10.

14. Produit conforme à la revendication 13, dans lequel le furoate de mométasone se trouve en une quantité d'à peu près 50 à 400 µg.

15. Produit conforme à la revendication 14, dans lequel le furoate de mométasone se trouve en une quantité d'à peu près 100 µg.

16. Produit conforme à la revendication 14, dans lequel le furoate de mométasone se trouve en une quantité d'à peu près 200 µg.

17. Produit conforme à la revendication 14, dans lequel le furoate de mométasone se trouve en une quantité d'à peu près 400 µg.

18. Produit conforme à la revendication 13, dans lequel ladite formulation ne contient pas d'excipients supplémentaires, et dans lequel l'inhalateur-doseur émet, après sa mise en action, une dose contenant une quantité régulière de médicament.

19. Produit conforme à la revendication 13, avec lequel la proportion des fines particules dispensées après mise en action de l'inhalateur-doseur vaut à peu près de 55 à 85 %, et dans lequel lesdites fines particules ont une taille de moins d'environ 4,7 µm.

20. Produit conforme à la revendication 19, avec lequel la proportion des fines particules dispensées après mise en action de l'inhalateur-doseur vaut à peu près de 65 à 80 %, et dans lequel lesdites fines particules ont une taille de moins d'environ 4,7 µm.
